Europäisches Patentamt

European Patent Office

Office européen des brevets

(19)

(11) Publication number: **0 103 914**
**B1**

(12) **EUROPEAN PATENT SPECIFICATION**

(45) Date of publication of patent specification: **07.01.88**

(51) Int. Cl.⁴: **F 01 K 23/06, C 07 C 1/04**

(21) Application number: **83201079.7**

(22) Date of filing: **20.07.83**

(54) Process for the generation of power and the preparation of liquid hydrocarbons.

(30) Priority: **26.08.82 NL 8203337**

(43) Date of publication of application:
**28.03.84 Bulletin 84/13**

(45) Publication of the grant of the patent:
**07.01.88 Bulletin 88/01**

(84) Designated Contracting States:
**BE DE FR GB IT NL**

(56) References cited:
**GB-A- 631 682**
**GB-A-2 075 124**
**NL-A-7 413 678**
**US-A-3 986 349**

(73) Proprietor: **SHELL INTERNATIONALE
RESEARCH MAATSCHAPPIJ B.V.**
**Carel van Bylandtlaan 30**
**NL-2596 HR Den Haag (NL)**

(72) Inventor: **Sie, Swan Tiong**
**Badhuisweg 3**
**NL-1031 CM Amsterdam (NL)**

(74) Representative: **Aalbers, Onno et al**
**P.O. Box 302**
**NL-2501 CH The Hague (NL)**

Courier Press, Leamington Spa, England.

**Description**

The invention relates to a process for the generation of power and the preparation of liquid hydrocarbons by converting part of a mixture of carbon monoxide and hydrogen by means of a hydrocarbon synthesis carried out above 300°C, separating from the reaction product a liquid fraction which comprises the desired hydrocarbons and a gaseous fraction which, in addition to unconverted carbon monoxide and hydrogen, comprises light hydrocarbons formed during the synthesis and which latter fraction is combusted, using the combustion gas for generating power in a gas turbine, producing high pressure steam from water by using hot process gas and generating power in a steam turbine with the high pressure steam.

Such a process is known from US—A—3,986,349 according to which the combustion gas can be used either as hot process gas for generation of steam to be directed to a steam turbine, or be expanded in a gas turbine for power generation; additional power is generated by combusting at least a portion of said liquid hydrocarbons, forming a flue gas, passing said flue gas through an expansion turbine, and using the exhausted flue gas to generate additional steam for use in said steam turbine.

Furthermore, it is known from GB—A—2,075,124 to use one part of a fuel gas produced in a coal gasification plant in a combined cycle power plant containing a gas turbine and a steam generator and -turbine operating on the exhaust gas from the gas turbine; another part of the fuel gas is used to synthesize methanol which can be employed as fuel in the gas turbine or in a separate peak load gas turbine generator. Boiler feed water from the steam generator can be employed in the methanol synthesis plant to produce steam.

A much more effective solution to the problem of the utilization of heat may be found in combining the generation of power with high-temperature hydrocarbon synthesis and utilizing the high-grade waste heat of the hydrocarbon synthesis reaction for evaporating water in the production of steam as the feed for the steam turbine. By combining the two processes in such a way that on the one hand unconverted synthesis gas, together with gaseous by-products formed, is used as fuel for the gas turbine, whilst on the other hand waste heat from the hydrocarbon synthesis is used for producing high-pressure steam the drawbacks of both the known combined high-temperature hydrocarbon synthesis/gas turbine/steam turbine system and methanol synthesis/combined cycle power plant, i.e. less efficient heat recuperation and high-pressure steam production, are overcome.

Use of synthesized liquid hydrocarbons and methanol, respectively, as fuel in a gas turbine/steam generator combination to produce additional steam and/or power can thus be avoided, if desired.

The above-mentioned "high-temperature hydrocarbon synthesis" refers to a hydrocarbon synthesis which is carried out at a temperature above 300°C. The products of said hydrocarbon synthesis are aliphatic and/or aromatic hydrocarbons, but not oxygenates like methanol, although oxygenates may be intermediates in the production of hydrocarbons from synthesis gas.

The invention therefore relates to a process for the generation of power and the preparation of liquid hydrocarbons by converting part of a mixture of carbon monoxide and hydrogen by means of a hydrocarbon synthesis carried out above 300°C, separating from the reaction product a liquid fraction which comprises the desired hydrocarbons and a gaseous fraction which, in addition to unconverted carbon monoxide and hydrogen, comprises light hydrocarbons formed during the synthesis and which latter fraction is combusted, using the combustion gas for generating power in a gas turbine, producing high pressure steam from water by using hot process gas and generating power in a steam turbine with the high-pressure steam, characterized in that high pressure steam is produced by pre-heating and superheating of water using as hot process gas the exit gas of the gas turbine, that less than 70 %v of the mixture of carbon monoxide and hydrogen is converted in the hydrocarbon synthesis step and that all the reaction heat from the hydrocarbon synthesis is used in the vaporization stage of the steam production step.

The process proposed here is very suitable for handling both hydrogen-rich and low-hydrogen synthesis gases.

With a proper choice of catalyst the high-temperature hydrocarbon conversion allows a partial conversion of low-hydrogen synthesis gases into liquid hydrocarbons without the necessity of previously subjecting the synthesis gas to the water gas shift reaction as is often required in methanol synthesis. At equal pressures (for instance 30 bar) higher conversion can be achieved in the high-temperature hydrocarbon synthesis than in the methanol synthesis. The high-temperature hydrocarbon synthesis releases more heat (20—25% of the energy content of the synthesis gas) than the methanol synthesis. Since the former reaction takes place at a high temperature, this high-grade heat can be put to good use, viz. in the vaporization step during the production of high-pressure steam which can be utilized in the steam cycle of the power plant. For instance, the reaction heat which is released when the hydrocarbon synthesis is carried out at a temperature of 375—400°C can be very well used for producing steam of 350°C and 165 bar. The high-temperature hydrocarbon synthesis may yield in addition to liquid hydrocarbons, considerable quantities of gaseous hydrocarbons ($C_1$—$C_4$ paraffins). However, when the high-temperature hydrocarbon synthesis is used in combination with power generation, this presents no difficulties, since these gaseous hydrocarbons fully contribute to the energy content of the fuel for the power plant and—as far as the calorific value of this fuel is concerned—even make up to some content for the diluting effect of

$CO_2$ which may be present in the fuel for instance as a result of an internal water gas shift reaction occurring during the hydrocarbon synthesis starting from a low-hydrogen synthesis gas. So long as the gas remains combustible there is no objection to the presence of $CO_2$ since it can have the same function as excess air of restricting the combustion temperature, with the advantage that the $CO_2$ is already under pressure. Since many catalysts used in the high-temperature hydrocarbons synthesis have a much higher sulphur tolerance than several methanol synthesis catalysts, like a Cu/ZnO catalyst, the sulphur-removing process required in the hydrocarbon synthesis when sulphur-containing synthesis gases are used—such as those formed during the gasification of coal—will be of a much simpler nature. In the high-temperature hydrocarbon synthesis it is extremely difficult to attain a synthesis gas conversion of more than 70%v when using a space velocity which is acceptable in practice, but no recirculation. However, when the high-temperature hydrocarbon synthesis is used in combination with power generation, this presents no difficulties, since in this combination the synthesis gas is meant to be converted only partly.

Depending on the catalyst chosen, the high-temperature hydrocarbon synthesis may yield either substantially aromatic hydrocarbons, or substantially paraffinic hydrocarbons. If substantially aromatic hydrocarbons are to be prepared, preference is given to the use of a bifunctional catalyst combination comprising one or more metal components having catalytic activity for the conversion of a $H_2$/CO mixture into acyclic hydrocarbons and/or acyclic oxygen-containing organic compounds and a crystalline metal silicate of a special structure which is capable of catalysing the conversion of acyclic compounds into aromatic hydrocarbons. If substantially paraffinic hydrocarbons are to be prepared, preference is given to the use of an iron-containing catalyst having activity for the conversion of a $H_2$/CO mixture into paraffinic hydrocarbons.

As observed hereinbefore, the process according to the invention may be applied to both hydrogen-rich synthesis gases and low-hydrogen synthesis gases. The process according to the invention is of particular interest for application to low-hydrogen synthesis gases. Such synthesis gases having a $H_2$/CO molar ratio of less than 2,0 may very suitably be obtained by gasification of carbonaceous materials, such as brown coal, anthracite, coke, crude mineral oil and fractions thereof, as well as oils produced from tar sand and bituminous shale. The gasification is preferably carried out at a temperature of 900—1500°C and a pressure of 10—100 bar. In the process according to the invention the preferred starting material is a synthesis gas having a $H_2$/CO molar ratio higher than 0,25.

If in the process according to the invention the starting material is a low-hydrogen synthesis gas, the catalyst preferably used in the high-temperature hydrocarbon synthesis is a bi- or trifunctional catalyst the latter comprising, in addition to the components of a bifunctional catalyst, one or more metal components having CO-shift activity.

If the high-temperature hydrocarbon synthesis is carried out with the object of preparing substantially aromatic hydrocarbons, preference is given to the use of a bi- or trifunctional catalyst which, in addition to the metal components having catalytic activity, comprises a crystalline metal silicate of a special structure which is capable of catalysing the conversion of acyclic hydrocarbons and acyclic oxygen-containing organic compounds into aromatic hydrocarbons, the metal silicate being described e.g. in British patent specification No. 1,555,928 and British patent application No. 2,055,893.

If the aim of the high-temperature hydrocarbon synthesis is to prepare substantially paraffinic hydrocarbons while starting from a low-hydrogen synthesis gas, preference is given to the use of an iron-containing bifunctional catalyst or catalyst combination which, in addition to activity for the conversion of a $H_2$/CO mixture substantially into paraffinic hydrocarbons, has CO-shift activity. Preference is given to the use of a bifunctional catalyst prepared by impregnation and comprising iron supported on a carrier. Examples of such catalysts are $Fe/Mg/Al_2O_3$ and $Fe/Cr/SiO_2$ catalysts described in British patent specification No. 2,053,713 and No. 2,053,016, respectively.

Suitable conditions for carrying out the high temperature hydrocarbon synthesis are a temperature of 300—450°C, a pressure of 20—80 bar and a space velocity of 200—2000 l (S.T.P.) gas/l catalyst/hour. When the high temperature hydrocarbon synthesis is used for the preparation of substantially aromatic hydrocarbons, the following conditions are preferably used: a temperature of 325—400°C, a pressure of 30—60 bar and a space velocity of 300—3000 l (S.T.P.) gas/l catalyst/hour. When the high-temperature hydrocarbon synthesis is used for the preparation of substantially paraffinic hydrocarbons, the following conditions are preferably used: a temperature of 300—350°C, a pressure of 20—50 bar and a space velocity of 500—2000 l (S.T.P.) gas/l catalyst/hour. Conversion in the high-temperature hydrocarbon synthesis should be less than 70%v. Depending on the object pursued the conversion may vary within wide limits. Usually the conversion will be more than 10%v.

In general, the process according to the invention can be used for the generation of power and the preparation of liquid hydrocarbons from mixtures of carbon monoxide and hydrogen. The process is of particular interest for the generation of electricity and the preparation of synthetic gasoline, starting from low-hydrogen synthesis gas obtained in the high-pressure gasification of coal. Excellent catalysts for the conversion of low-hydrogen synthesis gases into synthetic gasolines are the afore-mentioned trifunctional catalyst combinations consisting of a mixture of catalyst capable of converting a $H_2$/CO mixture substantially into methanol and a crystalline metal silicate of a special structure capable of converting methanol into aromatic hydrocarbons. Since the use of these catalysts results in a liquid product which boils substantially in the gasoline range, this hydrocarbon synthesis is sometimes referred to as the "direct gasoline synthesis" (DGS). Hereinafter whenever mention is made of DGS, it will be to designate the direct gasoline synthesis described hereinbefore.

In power plants periods of normal load and periods of peak load succeed each other. In order to be able to meet the demand for power in peak load periods, supplementary fuel is supplied to the gas turbine. To this end the process according to the invention utilizes the liquid hydrocarbons prepared by way of the high-temperature hydrocarbon synthesis. Depending on the quality of the liquid hydrocarbons produced by high-temperature hydrocarbon synthesis, it may in some cases be more attractive to purchase the supplementary fuel needed to cope with peak loads elsewhere and to reserve the liquid hydrocarbons prepared in the process for other purposes. Such a situation may present itself, for instance, when the hydrocarbon synthesis is carried out as a DGS, which yields an extremely valuable product that can be put to excellent use as motor gasoline. However, as may be seen from the following, the process according to the invention is highly flexible, so that to a certain extent it is possible to meet fluctuations in power demand without having to fall back on supplementary fuel purchased from external sources or produced in situ.

The DGS produces a considerable quantity of reaction heat of a temperature level of 375—400°C. A reactor that is very suitable for carrying out the DGS is a reactor whose heat discharge takes place by means of internal cooling pipes in which the heat is absorbed by water which boils, for instance, at 350°C and 165 bar. If the synthesis reactor is situated on the same site as the power plant, this provides an excellent opportunity for integrating this steam system into the combined gas turbine-steam turbine system. Preferably, the DGS is carried out isothermally, so that it releases the heat at a very attractive constant temperature level. Therefore this heat is excellently suitable for providing the heat of vaporization during the production of high-pressure steam and the utilization of the heat present in the exhaust gas of the gas turbine can be principally restricted to heating the water and superheating the steam. Data of situations in which 50 and 100%, respectively, of the heat of vaporization is provided by the DGS show that the process may be carried out more efficiently. (See Tables A and B). In particular in the case where 100% of the heat of vaporization is provided by the DGS the countercurrent exchange of heat is virtually ideal, resulting in a maximum gain of heat from the exhaust gas and consequently very low stack temperatures, of 91 and 67°C at gas turbine outlet temperatures of 550 and 600°C, respectively. The almost maximum quantity of heat recovered from the exhaust gas and the heat produced by the synthesis reaction together become available as high-pressure superheated steam (165 bar, 520—550°C) which can be converted into power with good efficiency. Not only is in this way the reaction heat of the hydrocarbon synthesis—which usually becomes available as saturated steam—upgraded, but more steam is produced besides. As shown in Table B, the reaction heat of the DGS is transformed to an additional quantity of superheated high pressure steam with an apparent efficiency of about 170% and finally it is transformed to extra power with an energy efficiency of about 70%. This is almost three times as high as when the reaction heat would have been simply utilized for the production of saturated steam to be used for the generation of power in a separate steam cycle of relatively poor efficiency. Not only is the latter option inferior from an energetic point of view, it is more costly as well, since a separate steam cycle has to be added to the system.

**0 103 914**

TABLE A

Power generation from gas turbine exhaust gas, in which the reaction heat of a DGS supplies at least part of the heat of vaporization of the water

$\Delta T_{min}=30°C$

$c_p$ exhaust gas=1.118 kJ . kg$^{-1}$ . °C$^{-1}$

steam condensor: P=0.04 bar, T=29°C

| Gas turbine outlet temperature, °C | 550 | | 600 | |
|---|---|---|---|---|
| Pressure of steam, bar<br>Temperature of steam, °C | 165<br>520 | | 165<br>550 | |
| % of heat of vaporization<br>supplied by DGS | 50 | 100 | 50 | 100 |
| Steam produced, kg/kg exhaust<br>gas | 0.151 | 0.220 | 0.182 | 0.247 |
| Heat recovered, kJ/kg<br>exhaust gas | 419.6 | 512.9 | 521.1 | 596.5 |
| Temperature of stack gas, °C | 170 | 91 | 134 | 67 |
| Heat supplied by DGS, kJ/kg<br>exhaust gas | 67.6 | 197.0 | 81.5 | 221.3 |
| Total steam energy, kJ/kg<br>exhaust gas | 487.3 | 709.9 | 602.6 | 817.8 |
| Steam turbine efficiency, % | 40.5 | 40.5 | 41 | 41 |
| Effective power, kJ/kg<br>exhaust gas | 197.4 | 287.5 | 247.1 | 335.3 |

# 0 103 914

TABLE B

| Effect of integration of DGS into a combined gas turbine-steam turbine system on power generation in steam cycle | | | | | | |
|---|---|---|---|---|---|---|
| | Gas turbine outlet temperature 550°C $P_{steam}$=165 bar $T_{steam}$=520°C | | | Gas turbine outlet temperature 600°C $P_{steam}$=165 bar $T_{steam}$=550°C | | |
| % of heat of vaporization supplied by DGS | 0 | 50 | 100 | 0 | 50 | 100 |
| Temperature of stack gas, °C | 223 | 170 | 91 | 186 | 134 | 67 |
| Steam produced, kg/kg exhaust gas | 0.113 | 0.151 | 0.220 | 0.140 | 0.182 | 0.247 |
| Total steam energy, kJ/kg exhaust gas | 365.9 | 487.3 | 709.9 | 462.5 | 602.5 | 817.8 |
| Extra steam energy produced=$\Delta$S, kJ/kg exhaust gas | 0 | 121.4 | 341.0 | 0 | 140.1 | 355.3 |
| Heat supplied by DGS= G, kJ/kg exhaust gas | 0 | 67.6 | 197.0 | 0 | 81.5 | 221.3 |
| Apparent efficiency of utilization of DGS reaction heat=$\Delta$S/G, % | — | 179 | 175 | — | 172 | 169 |
| Extra power generated= $\Delta$E, kJ/kg exhaust gas | 0 | 49.2 | 139.2 | 0 | 57.5 | 152.0 |
| Efficiency of conversion DGS reaction heat into power=$\Delta$E/G, % | — | 73 | 71 | — | 71 | 69 |

As already observed hereinbefore, the process according to the invention has a high degree of flexibility, so that, within certain limits, it is possible to meet fluctuations in power demand. For in the process according to the invention an increase in demand for power can be excellently met by reduction of the conversion in the hydrocarbon synthesis, resulting in more synthesis gas becoming available for use as fuel gas for the generation of power. The necessary fluctuations in degree of conversion in the hydrocarbon synthesis are preferably achieved by feeding part of the synthesis gas into the gas turbine by way of a by-pass around the synthesis reactor. According as more synthesis gas is diverted, the space velocity in the reactor will decrease. This reduces the absolute quantity of synthesis gas converted and, consequently the conversion based on the total quantity of synthesis gas feed. Since the reactor temperature is largely controlled by the boiling water in the internal cooling pipes, a reduction in the gas flow rate will have but little effect on the reactor temperature. The invention is now elucidated with the aid of the following example.

Example

In order to assess the advantages of the integration of a DGS into a combined gas turbine-steam turbine system (CGSS), four schemes were elaborated describing the conversion of coal into nothing but electricity (A), into nothing but gasoline (D) and into both gasoline and electricity (B and C). In scheme B the same amount of electricity was generated as in scheme A, while the relative ratio between gasoline, energy and electricity were such that the reaction heat of the DGS supplied 100% of the heat of vaporization, whereas in scheme C only half this quantity of heat was supplied. The Table C was made with the aid of the following data:

Coal feed:
Ash content 12%w; moisture content 6.5%w; analysis of coal: 78.1%w C; 5.5%w H; 4.3%w S; 1.2%w N; 10.9%w O. Calorific value 31.6 MJ/kg moisture and ash free coal (MAF coal).

6

**Gasification:**

High-pressure gasification using a mixture of steam and oxygen. Temperature 1500°C; pressure 30 bar; residence time of coal in reactor: 0.55 s.

$O_2$/MAF coal=0.89 kg/kg.

$H_2O$/MAF coal=0.08 kg/kg.

Yield: 1.99 m$^3$ (S.T.P.) purified synthesis gas per kg MAF coal having the composition: 32.8%v $H_2$; 66.5 %v CO; 0.7%v $N_2$+Ar.

Calorific value of synthesis gas: 12 MJ/m$^3$ (S.T.P.). Energy needed for preparing oxygen and process steam is supplied by a waste heat boiler integrated into the coal gasifier.

**Hydrocarbon synthesis:**

DGS over a catalyst consisting of a mixture of a ZnO—$Cr_2O_3$ composition and a crystalline iron/aluminium silicate of a special structure, as disclosed in British patent specification No. 1,555,928.

P=30 bar, T=400°C.

$C_3$+ selectivity: 95%w on $C_1$+.

$C_5$+ selectivity: 80%w on $C_1$+.

**Gas turbine:**

Efficiency expressed as specific energy consumption: 11290 kJ/kWh.

Inlet temperature: 1130°C.

Outlet temperature: 552°C.

Pressure ratio ±14.2:1.

Air flow rate 12.35 t/MWh.

**Exhaust gas boiler:**

Preheater+superheater ΔT=30°C, minimally.

**Steam turbine:**

$P_{steam}$=165 bar; $T_{steam}$=520°C.

$P_{condensor}$=0.04 bar; $T_{condensor}$=29°C.

Energy conversion efficiency: 40.5%.

In Table C the most significant results of the schemes are given. In scheme B about 35% more coal is gasified than in scheme A and about half of the synthesis gas is converted into gasoline. Although as compared with scheme A, less CO and $H_2$ is left over for use as fuel for the gas turbine, the same amount of electric power is produced. In case of gasoline production of approximately the same energy content the total effective energy produced by scheme B is almost redoubled. The thermal efficiency of the coal conversion has been increased from less than 40% to over 50%.

Comparison of scheme B with scheme D and scheme A shows that a quantity of 173 t/h of coal would be needed to produce about the same quantities of electricity and gasoline as produced in scheme B starting from 134 t/h. This clearly shows the advantage to be obtained from the integration of the two processes.

As regards meeting demand peaks, the following may be observed. If the integrated process is constructed in such a way that scheme B (about 50% synthesis gas conversion in DGS) corresponds with the base load, an increase in electricity demand can be met within certain limits by reduction of the conversion, so that more gas is available for use as fuel for the CGSS. In scheme C the same quantity of coal (134.5 t/h) is supplied to the gasifier as in scheme B, but conversion in the DGS is reduced to 24%. Consequently the quantity of electricity produced grows (from 323 to 375 MW). As is seen from Table C, virtually the entire increase in electric power occurs in the section of the CGSS which is connected with the gas turbine, whereas the section connected with the steam production is barely involved. This is very fortunate, since rapid response and relatively low cost make gas turbines eminently suitable for meeting demand peaks. Thus, by installing sufficient gas turbine capacity, fluctuations in the demand for electricity can be met to a certain extent, while the most important and most costly parts used in the conversion of coal to electricity are constantly operated at full capacity. The advantages of integration are still quite substantial at a low degree of synthesis gas conversion.

7

# 0 103 914

TABLE C

| Scheme | A<br>Only electricity | B<br>Gasoline and electricity,<br>100% of heat of vaporization of water supplied by DGS | C<br>Gasoline and electricity,<br>50% of heat of vaporization of water supplied by DGS | D<br>Only gasoline |
|---|---|---|---|---|
| Coal supply, t/h MAF<br>MW equivalent | 100.0<br>878 | 134.5<br>1180 | 134.5<br>1180 | 73.4<br>644 |
| Synthesis gas produced,<br>$10^3$ m³/h (S.T.P.)<br>MW equivalent | 199<br>663 | 268<br>892 | 268<br>892 | 146<br>487 |
| Synthesis gas converted in synthesis step, % | — | 51.8 | 24.0 | 95.0 |
| $C_5^+$ Gasoline produced, t/h<br>MW equivalent | —<br>— | 23.1<br>276 | 11.2<br>134 | 23.1<br>276 |
| Electricity generated, MW<br>Gas turbine<br>Steam turbine<br>————+<br>Total | 212<br>111<br><br>323 | 160<br>163<br><br>323 | 221<br>154<br><br>375 | —<br>—<br><br>— |
| Total useful energy produced, MW | 323 | 599 | 509 | 276 |
| Thermal efficiency (synthesis gas to useful products), % | 48.7 | 67.2 | 57.1 | 56.9 |
| Thermal efficiency (coal to useful products), % | 36.9 | 50.8 | 43.1 | 42.9 |

**Claims**

1. A process for the generation of power and the preparation of liquid hydrocarbons by converting part of a mixture of carbon monoxide and hydrogen by means of a hydrocarbon synthesis carried out above 300°C, separating from the reaction product a liquid fraction which comprises the desired hydrocarbons and a gaseous fraction which, in addition to unconverted carbon monoxide and hydrogen, comprises light hydrocarbons formed during the synthesis and which latter fraction is combusted, using the combustion gas for generating power in a gas turbine, producing high pressure steam from water by using hot process gas and generating power in a steam turbine with the high-pressure steam, characterized in that said high pressure steam is produced by pre-heating and superheating of water using as hot process gas the exit gas of the gas-turbine, that less than 70%v of the mixture of carbon monoxide and hydrogen is converted in the hydrocarbon synthesis step and that all the reaction heat from the hydrocarbon synthesis is used in the vaporization stage of the steam production step.

2. A process as claimed in claim 1, characterized in that the hydrocarbon synthesis is carried out at a temperature of 300—450°C, a pressure of 20—80 bar and a space velocity of 200—2000 l (S.T.P.) gas/l catalyst/hour.

3. A process as claimed in claim 1 or 2, characterized in that it is applied to a synthesis gas having a $H_2$/CO molar ratio in the range between 0,25 and 2,0.

4. A process as claimed in claim 3, characterized in that it is applied to a synthesis gas obtained in the gasification of coal at a temperature of 900—1500°C and a pressure of 10—100 bar.

5. A process as claimed in any one of claims 1—4, characterized in that an increase in demand for power is met by reduction of the conversion in the hydrocarbon synthesis step, resulting in more synthesis gas becoming available for use as fuel gas for the generation of power.

8

**Patentansprüche**

1. Ein Verfahren zur Erzeugung von Energie und zur Herstellung flüssiger Kohlenwasserstoffe durch Umwandlung einer Mischung aus Kohlenmonoxid und Wasserstoff mittels einer bei oberhalb 300°C durchgeführten Kohlenwasserstoffsynthese, Abtrennen einer flüssigen, die gewünschten Kohlenwasserstoffe enthaltenden Fraktion und einer gasförmigen Fraktion aus dem Reaktionsprodukt, welche außer dem nicht umgesetzten Kohlenmonoxid bzw. Wasserstoff leichte, während der Synthese gebildete Kohlenwasserstoffe umfaßt, wobei die zuletzt gennante Fraktion verbrannt wird, das Verbrennungsgas zur Erzeugung von Energie in einer Gasturbine verwendet wird, ferner unter Verwendung von heißem Verfahrensgas aus Wasser Hochdruckdampf erzeugt und mittels dieses Hochdruckdampfes in einer Dampfturbine Energie erzeugt wird, dadurch gekennzeichnet, daß besagter Hochdruckdampf durch Vorerhitzen und Übererhitzen von Wasser unter Verwendung des Auslaßgases der Gasturbine als heißes Verfahrensgas erzeugt wird, daß weniger als 70 Volumenprozent der Mischung aus Kohlenmonoxid und Wasserstoff in der Syntheseverfahrensstufe in Kohlenwasserstoffe umgewandelt werden und daß die gesamte Reaktionswärme der Kohlenwasserstoffsynthese in der Verdampfungsstufe des Dampferzeugungsverfahrensschrittes eingesetzt wird.

2. Ein Verfahren wie in Anspruch 1 beansprucht, dadurch gekennzeichnet, daß die Kohlenwasserstoffsynthese bei einer Temperatur von 300 bis 450°C, einem Druck von 20 bis 80 bar und einer Raumgeschwindigkeit von 200 bis 2000 NI (S.T.P.) Gas/Liter Katalysator/Stunde durchgeführt wird.

3. Ein Verfahren wie in Anspruch 1 oder 2 beansprucht, dadurch gekennzeichnet, daß es bei einem Synthesegas mit einem Molverhältnis von $H_2$:CO im Bereich zwischen 0,25 und 2,0 angewendet wird.

4. Ein Verfahren wie in Anspruch 3 beansprucht, dadurch gekennzeichnet, daß es bei einem Synthesegas angewendet wird, welches durch Vergasung von Kohle bei einer Temperatur von 900 bis 1500°C und einem Druck von 10 bis 100 bar erhalten worden ist.

5. Ein Verfahren wie in irgendeinem der Ansprüche 1 bis 4 beansprucht, dadurch gekennzeichnet, daß ein steigender Energiebedarf durch Verminderung der Umwandlung in der Kohlenwasserstoffsynthesestufe gedeckt wird, wodurch mehr Synthesegas für die Verwendung als Brennstoffgas bei der Erzeugung von Energie zur Verfügung steht.

**Revendications**

1. Procédé de production d'énergie et de préparation d'hydrocarbures liquides par transformation d'une partie d'un mélange de monoxyde de carbone et d'hydrogène au moyen d'une synthèse d'hydrocarbure conduite au-dessus de 300°C, séparation d'avec le produit réactionnel d'une fraction liquide qui comprend les hydrocarbures désirés et d'une fraction gazeuse qui, outre le monoxyde de carbone et l'hydrogène non transformés, comprend des hydrocarbures légers formés pendant la synthèse et laquelle dernière fraction est mise à la combustion, utilisation du gaz de combustion pour produire de l'énergie dans une turbine à gaz, production de vapeur haute pression à partir de l'eau en utilisant du gaz de traitement à chaud et production d'énergie dans une turbine à vapeur avec la vapeur haute pression, caractérisé en ce que ladite vapeur haute pression est produite par préchauffage et surchauffe de l'eau en utilisant comme gaz de traitement chaud le gaz de sortie de la turbine à gaz, en ce que moins de 70%v du mélange de monoxyde de carbone et d'hydrogène est transformé dans l'étape de synthèse des hydrocarbures et en ce que toute la chaleur de réaction de la synthèse des hydrocarbures est utilisée dans le stade de vaporisation de l'étape de production de vapeur.

2. Procédé selon la revendication 1, caractérisé en ce que la synthèse des hydrocarbures est conduite à une température de 300—450°C, une pression de 20—80 bar et une vitesse spatiale de 200—2000 l (P.T.S.) de gaz/l catalyseur/heure.

3. Procédé selon la revendication 1 ou 2, caractérisé en ce qu'il est appliqué à un gaz de synthèse ayant un rapport molaire $H_2CO$ dans un intervalle compris entre 0,25 et 2,0.

4. Procédé selon la revendication 3, caractérisé en ce qu'il est appliqué à un gaz de synthèse obtenu dans la gazéification du charbon à une température de 900—1500°C et une pression de 10—100 bar.

5. Procédé selon l'une quelconque des revendications 1—4, caractérisé en ce qu'on répond à un accroissement de la demande d'énergie par une réduction de la transformation dans l'étape de synthèse des hydrocarbures, ce qui about à rendre disponible une plus grande quantité de gaz de synthèse pour utilisation comme gaz combustible pour la production d'énergie.